# EUROPEAN PATENT APPLICATION

(11) **EP 3 929 963 A2**
(43) Date of publication of application: **29.12.2021**
(21) Application number: 21181109.6
(22) Date of filing: 23.06.2021
(51) Int. Cl.: H01J 61/16, A61L 2/10, H01J 61/30, H01J 61/36, H01J 65/04

(54) **ULTRAVIOLET EXCIMER LAMP SYSTEMS AND METHODS**

(30) Priority: 23.06.2020 US 202063042833 P; 21.06.2021 US 202117352875
(71) Applicant: The Boeing Company, Chicago, IL 60606-2016 (US)
(72) Inventor: CALLAHAN, Kevin S., CHCAGO, 60606-2016 (US); BROCKSCHMIDT, Arthur E., CHCAGO, 60606-2016 (US)
(74) Representative: Plasseraud IP

(57) **Abstract**

An excimer lamp 100 includes a first electrode 110, a dielectric plate 120, and a second electrode 130. The dielectric plate has a first side 122 and a second side 124 opposite the first side. The dielectric plate is spaced a distance from the first electrode to define a volume 150 configured to hold a gas. The first side of the dielectric plate is oriented toward the first electrode. The second electrode is oriented toward the dielectric plate, wherein the dielectric plate is interposed between the first electrode and the second electrode.

## Description

### FIELD OF THE DISCLOSURE

Embodiments of the present disclosure generally relate to excimer lamps, such as may be used to sanitize structures and areas within vehicles or other enclosed spaces, and more particularly to systems and methods of providing such lamps in convenient or customizable shapes.

### BACKGROUND OF THE DISCLOSURE

Vehicles such as commercial aircraft are used to transport passengers between various locations. Systems are currently being developed to disinfect or otherwise sanitize surfaces within aircraft, for example, that use ultraviolet (UV) light. UV light may also be used in other applications besides aircraft to disinfect or sanitize surfaces or objects.

For example, UV light around 222 nanometers wavelength may be utilized. However, known lamps that produce such wavelengths are of a fixed cylindrical shape or a fixed flat shape. Such shapes may be unwieldy or difficult to integrate into aircraft or other environments.

### SUMMARY OF THE DISCLOSURE

A need exists for a system and a method for providing UV lamps for sanitizing that are shaped to match a given environment or application.

With those needs in mind, certain embodiments of the present disclosure provide an excimer lamp that includes a first electrode, a dielectric plate, and a second electrode. The dielectric plate has a first side and a second side opposite the first side. The dielectric plate is spaced a distance from the first electrode to define a volume configured to hold a gas. The first side of the dielectric plate is oriented toward the first electrode. The second electrode is oriented toward the dielectric plate, wherein the dielectric plate is interposed between the first electrode and the second electrode.

Certain embodiments of the present disclosure provide a method for assembling an excimer lamp. The method includes disposing a first electrode proximate a frame having a cavity. The method also includes positioning a dielectric plate a distance from the first electrode. The dielectric plate has a first side and a second side opposite the first side, and is spaced a distance from the first electrode to define a volume configured to hold a gas. The first side of the dielectric plate is oriented toward the first electrode. Further, the method includes positioning a second electrode oriented toward the dielectric plate, wherein the dielectric plate is interposed between the first electrode and the second electrode. Also, the method includes supplying the volume with the gas.

### BRIEF DESCRIPTION OF THE DRAWINGS

Figure 1 illustrates a schematic view of an excimer lamp, according to an embodiment of the present disclosure.
Figure 2 illustrates views of a disc shaped lamp, according to an embodiment of the present disclosure.
Figure 3 illustrates a perspective view of a shaped lamp, according to an embodiment of the present disclosure.
Figure 4 illustrates a side view of a lamp, according to an embodiment of the present disclosure.
Figure 5 illustrates views of a lamp having a conductive frame, according to an embodiment of the present disclosure.
Figure 6 illustrates views of a lamp having an insulative frame, according to an embodiment of the present disclosure.
Figure 7 illustrates a flowchart of a method, according to an embodiment of the present disclosure.
Figure 8 illustrates a perspective front view of an aircraft, according to an embodiment of the present disclosure.
Figure 9A illustrates a top plan view of an internal cabin of an aircraft, according to an embodiment of the present disclosure.
Figure 9B illustrates a top plan view of an internal cabin of an aircraft, according to an embodiment of the present disclosure.
Figure 10 illustrates a perspective interior view of an internal cabin of an aircraft, according to an embodiment of the present disclosure.
Figure 11 illustrates a perspective internal view of a lavatory within an internal cabin of an aircraft.

### DETAILED DESCRIPTION OF THE DISCLOSURE

The foregoing summary, as well as the following detailed description of certain embodiments will be better understood when read in conjunction with the appended drawings. As used herein, an element or step recited in the singular and preceded by the word "a" or "an" should be understood as not necessarily excluding the plural of the elements or steps. Further, references to "one embodiment" are not intended to be interpreted as excluding the existence of additional embodiments that also incorporate the recited features. Moreover, unless explicitly stated to the contrary, embodiments "comprising" or "having" an element or a plurality of elements having a particular condition can include additional elements not having that condition.

Certain embodiments of the present disclosure provide a 222 nm UV lamp having a shape adapted to the interior of an aircraft (or other environment), and/or a method to manufacture such a lamp. For example, a conductive frame (or non-conductive frame supporting a conductive electrode) is provided in various embodiments in a desired shape to complement or correspond to a target environment. Additionally, a quartz (or other dielectric) plate in a matching shape is disposed on top of the frame, with a conductive mesh disposed on the quartz plate. In some embodiments, a band pass filter material is also embedded or mounted to the plate. A sealing mechanism (e.g., o-ring, clip) is utilized to seal the quartz plate to the frame to maintain gas in the frame for operation of the lamp. A sealable tube may be used to access an interior chamber defined by the frame to fill with a gas (e.g., Krypton Chlorine).

Various embodiments provide excimer lamps (and/or methods for making such lamps) in shapes that are adapted to a target environment, such as the architecture and appearance of an airplane interior design. Accordingly, UV light from the lamp may be integrated into the curvatures of the aircraft interior (or other target area) for use for disinfecting surfaces and air in the target area. In some embodiments, a band pass filter is utilized to provide safe and effective sanitization at a desired wavelength (or range of wavelengths). It may be noted that, alternatively to aircraft, various embodiments may be used in conjunction with, for example, other types of vehicles, or, as another example, commercial buildings.

Various embodiments provide a frame that may be machined or cast in a desired shape. The frame may be conductive, or, alternatively, non-conductive with a conductive electrode mounted to the frame. A transparent covering (e.g., quartz glass plate, fused silica glass plate) may be made by, for example, blow molding, and joined to the frame. The frame in various embodiments is made of a material having a thermal coefficient of expansion that matches or corresponds to the cover. The cover includes a conductive mesh grid. The conductive mesh grid may be formed, for example, by screen printing conductive ink on a surface of the cover, or, as another example, using a woven metal textile of thin conductive wires. The mesh may be laser cut. In various embodiments, the conductive mesh grid may be in a decorative pattern. The conductive mesh acts as an electrode. Optionally, a band pass filter material may be applied to the cover. As another option, an additional transparent plate may be positioned on the cover, with the conductive mesh sandwiched between the cover and the additional plate.

The cover (e.g., quartz plate) may be joined or coupled to the frame using one or more of an adhesive, an o-ring, or a clip. The frame in various embodiments includes or is associated with a tube used to vacuum fill a chamber of the frame with a gas such as Krypton Chlorine gas (e.g., 45 parts Krypton and 1 part Chlorine). The tube may be crimped to seal after filling. In some embodiments, the frame may be coated with a material (e.g., enamel) to protect against corrosion, and/or a reflector (e.g., made of aluminum) may be attached to the frame.

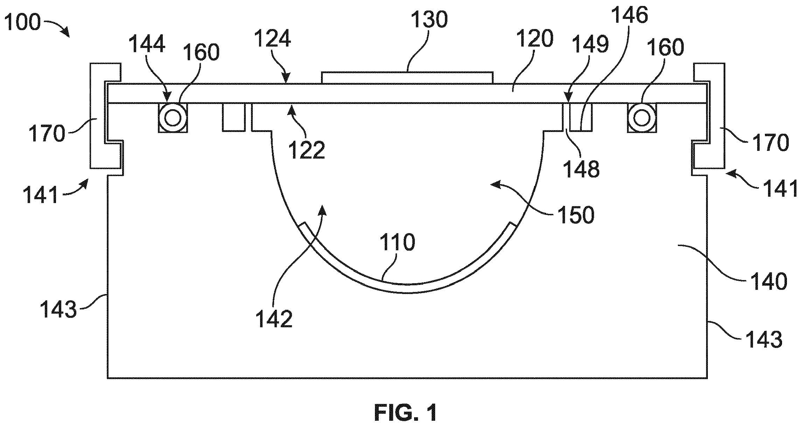

Figure 1 illustrates a block schematic view of an excimer lamp 100. The excimer lamp in the illustrated embodiment is configured to emit UV light (e.g., at a wavelength of about 222 nm). The depicted excimer lamp 100 includes a first electrode 110, a dielectric plate 120, and a second electrode 130.

Generally, a gas (e.g., a gas mixture including Krypton and Chlorine or Chloride) is maintained in a volume between the electrodes, and a current is applied to the electrodes to cause the gas to emit UV light. The depicted example includes a frame 140, which in some embodiments provides the first electrode 110, and in other embodiments supports the first electrode 110. Optionally, a second, protective plate (not shown in Figure 1) may be placed above the second electrode 130.

The depicted frame 140 supports the dielectric plate 120 as shown in Figure 1. For example, the frame may support the dielectric plate 120 circumferentially at or near a perimeter of the dielectric plate 120. The frame 140 includes a cavity 142. The cavity 142 in various embodiments may be shaped (e.g., parabolically) to help focus light or provide a desired light distribution of light emitted by the excimer lamp 100.

The dielectric plate 120 is made in various embodiments from a dielectric material that is transparent. For example, the dielectric plate 120 may be made of quartz glass, or, as another example, fused silica. As seen in Figure 1, the example dielectric plate 120 has a first side 122 and a second side 124. The second side 124 is opposite the first side 122. The first side 122 of the dielectric plate 120 is oriented toward the first electrode 110, and the second side 124 of the dielectric plate 120 is oriented toward the second electrode 130. The dielectric plate 120 is spaced a distance from the first electrode 110 to define a volume 150 configured to hold a gas. In the illustrated embodiment, the volume 150 is defined between the surface of the cavity 142 and the first side 122 of the dielectric plate 120.

The second electrode 130 is oriented toward the dielectric plate 120, with the dielectric plate 120 interposed between the first electrode 110 and the second electrode 130. The second electrode 120 in various embodiments is disposed on or formed on the second side 124 of the dielectric plate 120.

For example, the second electrode 130 in various embodiments is formed from a silk screened conductive ink that is printed on the second side 124 of the dielectric plate 120. As another example, the second electrode 130 in various embodiments is formed of a conductive mesh that is stretched over or otherwise joined to the second side 124 of the dielectric plate 120.

The frame 140 in various embodiments includes adaptations to help maintain a seal between the frame 140 and the dielectric plate 120 to maintain gas in the cavity 142. For example, in the depicted environment, the frame 140 includes a groove 144. The groove 144 extends circumferentially around the frame 140, and extends into an upper surface of the frame 140 that is configured to support the dielectric plate 120. Further, the excimer lamp 100 comprises an o-ring 160 that is disposed in the groove 144, and interposed between the dielectric plate 120 and the frame 140. The o-ring 160 provides a seal to maintain gas within the excimer lamp 100.

The depicted example frame 140 also includes a recessed surface 146 and a projection 148. The recessed surface 148 is located proximate the groove 144, laterally or radially inward of the groove 144 (e.g., located more closely to a center of the excimer lamp 100 than is the groove 144). The projection 148 extends from the recessed surface 146 toward the dielectric plate 120, and includes a land 149 that is configured to contact the dielectric plate 120 when the dielectric plate 120 is joined to the frame 140. The projection 148 helps protect the o-ring from UV light exposure and/or materials in the cavity 142.

The dielectric plate 120 may be mechanically secured to the frame 140. For example, in the illustrated embodiment, the frame 140 includes a notch 141 that extends circumferentially around a side 143 of the frame 140. Further, the depicted example excimer lamp 100 includes a band 170 mounted to the frame 140 in the notch 141. The band 170 acts as a clip to secure the dielectric plate 120 to the frame 140. In some embodiments, a series of discrete clips may be used to secure the dielectric plate 120 to the frame 140. As another option, the dielectric plate 120 may be brazed or otherwise sealingly joined to the frame 140.

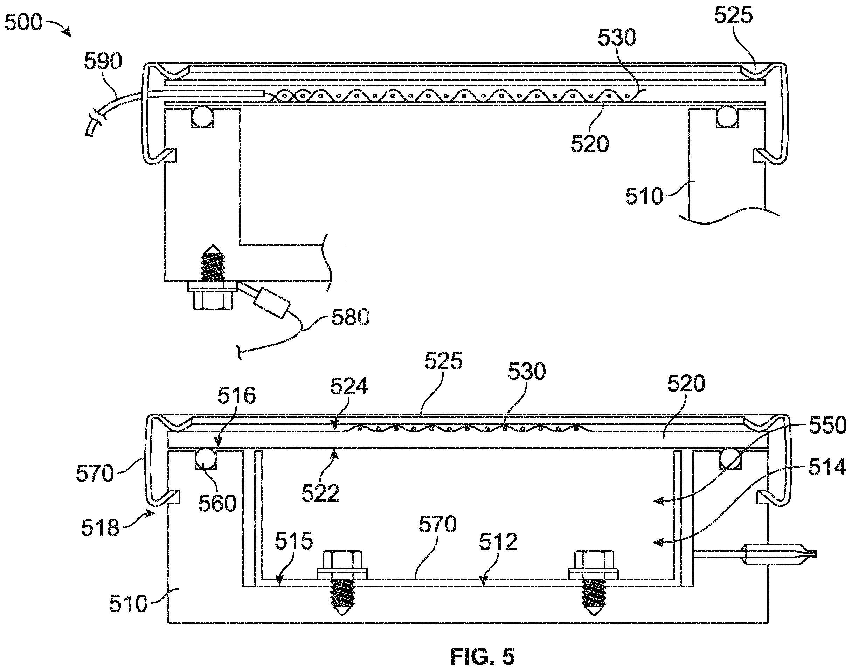

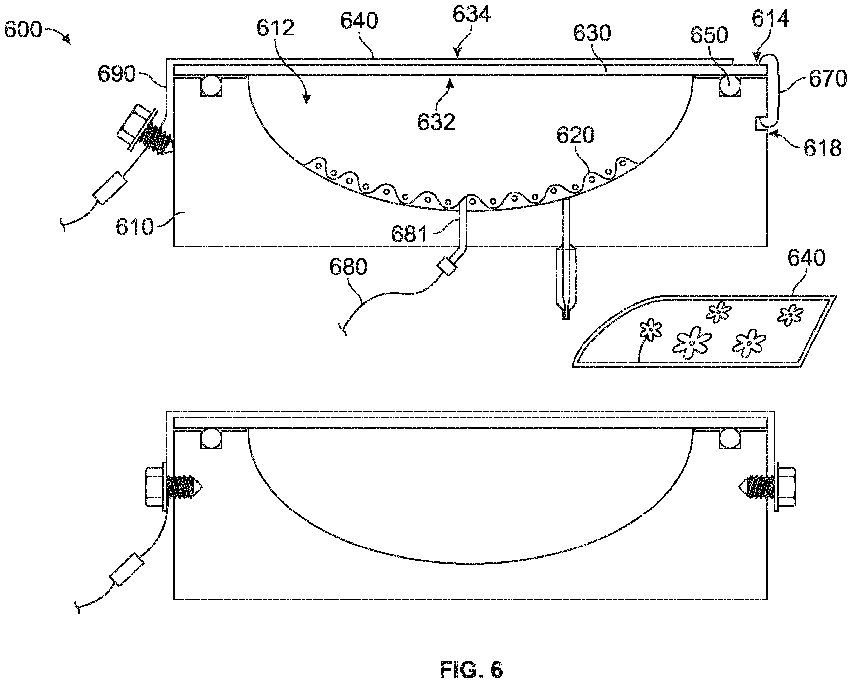

In some embodiments, the frame 140 is formed of an electrically conductive material, and the first electrode 110 is integrally formed with the frame 140. (See, e.g., Figure 5 and related discussion.) In some embodiments, the frame 140 is formed of an electrically insulative material, and the first electrode 110 is supported by the frame 140. (See, e.g., Figure 6 and related discussion.)

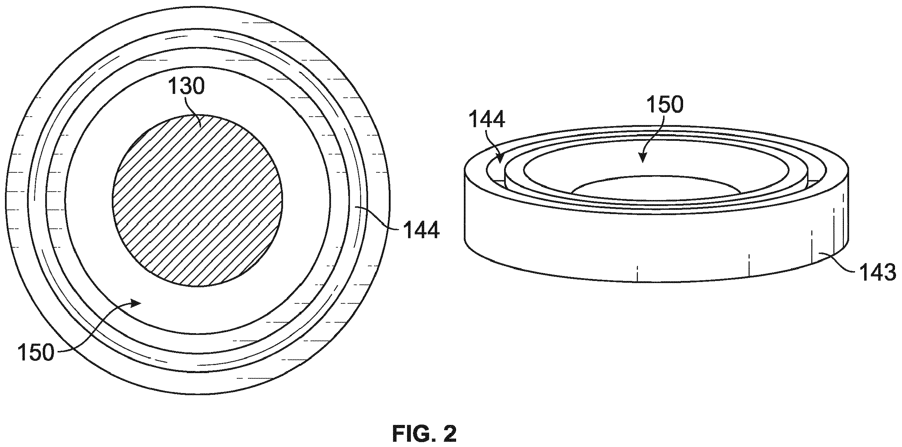

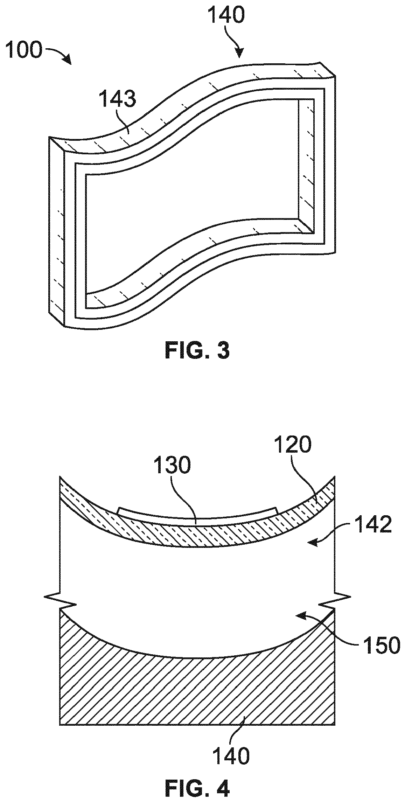

Due to the structure of the excimer lamp 100, the excimer lamp 100 may be formed in a variety of shapes, for example by forming the frame 140 and/or the dielectric plate 120 to a desired shape. Figure 2 illustrates an example where the excimer lamp 100 has a disc shape, with the second electrode 130 formed as a disc at the center of the excimer lamp 100. As another example, Figure 3 provides a perspective view of an example excimer lamp 100 that has a shape formed to conform to an existing environment.

It may further be noted that the dielectric plate need not be flat. For example, Figure 4 provides a side sectional view of an example of the excimer lamp where the dielectric plate 120 and the second electrode 130 have a concave shape. For example, the concave shape may correspond to an object to be irradiated by the excimer lamp 100. A simple concave curve is shown in Figure 4 for ease and clarity of illustration. However, it should be noted that more complex shapes, including changes of curvature, convex portions, flat portions, angled portions, or the like, may be used in other embodiments, for example to more closely match a particular shape of an object to be irradiated. Further, it may be noted that the cavity of the frame 140 may be shaped to match, correspond to, or complement the shape of the dielectric plate 120. In the illustrated embodiment, the frame 140 is shaped to provide a curved surface similar to the curved surface of the dielectric plate 120 to provide a uniform distance between electrodes. For example, as seen in Figure 4, the upper surface of the cavity and lower surface of the dielectric plate 120 illustrate complementary non-planar surfaces that have a uniform offset distance.

As discussed above, in some embodiments the frame 140 is made of an electrically conductive material. Figure 5 provides views of an excimer lamp 500 that includes a frame 510 made of a conductive material. It may be noted that the excimer lamp 500 may incorporate one or more aspects of the excimer lamp 100 discussed herein, and provides an example of the excimer lamp 100.

The excimer lamp 500 includes a frame 510, a dielectric plate 520, and a second electrode 530. The frame 510 is made of a conductive material and defines a first electrode 512. The frame 510 may be made of a material (e.g., Kovar, Invar) that has a thermal coefficient of expansion similar to the dielectric plate 520 to reduce or eliminate stresses or strains caused by changing dimensions of the components under changing temperature conditions. The frame 510 has a cavity 514.

The dielectric plate 520 has a first side 522, and a second side 524 that is opposite the first side 522, with the first side 522 of the dielectric plate 520 oriented toward the frame 510. The dielectric plate 520 is spaced a distance from the frame 510 to define a volume 550 bounded by the first side 522 of the dielectric plate 520 and the surface of the cavity 514. The volume 550 is configured to hold a gas. The second electrode 530 is oriented toward the dielectric plate 520 (e.g., toward the second side 524 of the dielectric plate 520), with the dielectric plate 520 interposed between the frame 516 and the second electrode 530.

Generally, a seal may be maintained between the dielectric plate 520 and the frame 510 to keep the gas within the volume 550. For example, the frame 510 in various embodiments includes a groove 516 that extends circumferentially (i.e., around a perimeter) of the frame 510. An o-ring 560 of the excimer lamp 500 is disposed in the groove 516, and interposed between the dielectric plate 520 and the frame 510 to provide a seal. In some embodiments, a hollow metal o-ring may be used to seal the dielectric plate 520 to the conductive frame 510 (e.g., in conjunction with brazing).

The illustrated example frame 510 also includes a notch 518 that extends circumferentially around a side 519 of the frame 510, with the excimer lamp 500 also include a band 570 mounted to the frame 510 in the notch 518. The band 570 helps secure the dielectric plate 520 to the frame 510. As additional examples of sealing and/or joining between the frame 510 and dielectric plate 520, a sealant may be applied, or the frame 510 and dielectric plate 520 may be brazed together.

In various embodiments, a reflector or reflector surface may be provided to the surface of the cavity 514. In the illustrated example, the excimer lamp 500 includes a reflector 570 that is mounted to a surface 515 of the cavity 514 of the frame 510. For example, the reflector 570 may be made of polished aluminum, and be fastened to the frame 510 with threaded fasteners. Alternatively, the surface 515 may be coated. For example, the surface 515 may be coated with an enamel or other material to help protect against arc pitting.

In some embodiments, the excimer lamp 500 (or excimer lamps 100, 600) includes a protective plate 525 that is located proximate the second electrode 530. The second electrode 530 is interposed between the dielectric plate 520 and the protective plate 525. The protective plate 525 in various embodiments is made of the same material as the dielectric plate 520, and is thicker than the dielectric plate 520.

In the illustrated example, a ground conductor 580 is coupled to the first electrode 512 (i.e., frame 510). For example, the ground conductor 580 may be coupled to the frame 510 via a crimped eyelet.

Also in the illustrated example, a positive conductor 590 is coupled to the second electrode 530. In the illustrated example, for instance, the positive conductor 590 includes an insulated high voltage lead that has a stripped conductor end that soldered or crimped on to the second electrode 530.

A fill tube may be used to introduce gas into the volume 550. The fill tube for example may be made of Kovar or Invar that is pressed into place in a corresponding opening of the frame 510 using an interference fit. As another example, the fill tube may be brazed in place. After the volume 550 is filled, an end of the fill tube may be crimped to maintain the gas within the volume 550.

As discussed above, in some embodiments the frame 140 is made of an electrically insulating material. Figure 6 provides views of an excimer lamp 600 that includes a frame 610 made of an insulative material. It may be noted that the excimer lamp 600 may incorporate one or more aspects of the excimer lamp 100 discussed herein, and provides an example of the excimer lamp 100.

The excimer lamp 600 includes a frame 610 made of an insulative material and having a cavity 612, and a first electrode 620 made of a conductive material and mounted to the frame 610. The excimer lamp 600 also includes a dielectric plate 630 (e.g., quartz or fused silica glass) that has a first side 632 and a second side 634, with the second side 634 opposite the first side 632, and the first side 632 oriented toward the frame 610. The dielectric plate 630 is spaced a distance from the first electrode 620 to define a volume 613 (between a surface of the cavity 612 and the first side 632 of the dielectric plate 630) that is configured to hold a gas.

The excimer lamp 600 also includes a second electrode 640 that is oriented toward the dielectric plate 630. The dielectric plate 630 is interposed between the first electrode 620 and the second electrode 640. The second electrode 640 may be formed on or mounted to the second side 634 of the dielectric plate 620. As one example, the second electrode 640 may be formed of a decorative silk screen 632 that is printed on the second side 634 of the dielectric plate 630. As another example, the second electrode 640 may include a conductive mesh 634 that is stretched over the second side 634 of the dielectric plate 630.

The electrodes are coupled to a power supply to cause the gas to emit light. In the example depicted in Figure 6, the first electrode 620 is coupled to a positive conductor 680, and the second electrode 640 is coupled to a ground conductor 690. For example, a positive electrode 681 may be pressed into the frame 610 with sealant, and a wire crimped to the positive electrode 681. For example where the frame 610 is made of Teflon, an epoxy may be used for sealing. For examples where the frame 610 is ceramic, an active metal braze (e.g., titanium alloyed with the braze material) may be used. In some embodiments, a fill tube may be pressed into place with sealant and used to fill the cavity 612 with gas.

In the depicted example, a seal is provided between the frame 610 and dielectric plate 630 via an o-ring 650. The o-ring 650 is disposed in a groove 614 of the frame 610 that extends circumferentially around the frame 610. Also, the illustrated example frame 610 also includes a notch 618 that extends circumferentially around a side 619 of the frame 610, with the excimer lamp 600 also include a band 670 mounted to the frame 610 in the notch 618. The band 670 helps secure the dielectric plate 630 to the frame 610. As additional examples of sealing and/or joining between the frame 610 and dielectric plate 630, a sealant may be applied, or the frame 610 and dielectric plate 630 may be brazed together.

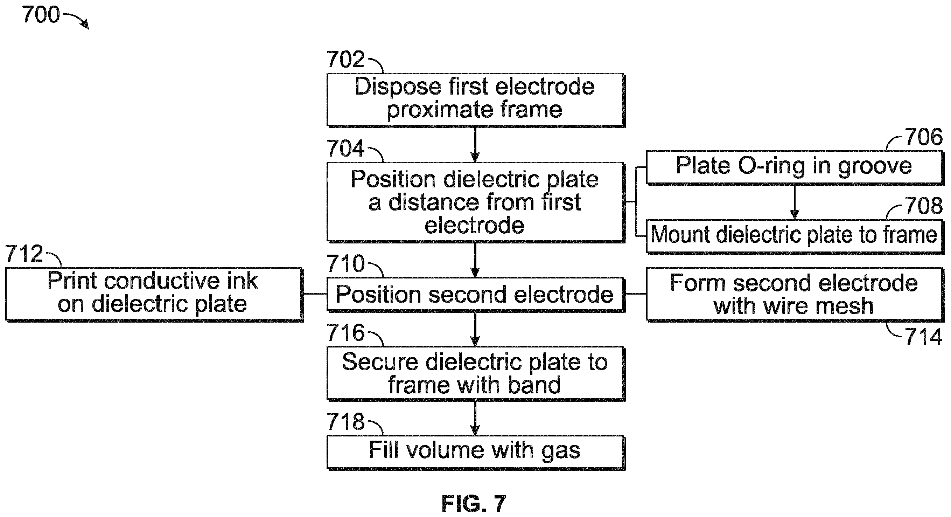

Figure 7 provides a flowchart of a method 700 for assembling an excimer lamp (e.g., excimer lamps 100, 500, 600). The method 700 in various embodiments utilizes and/or provides one or more aspects discussed above in connection with excimer lamps 100, 500, 600. It may be noted that steps may be added or omitted in various embodiments, and/or various steps may be performed in a different order than shown in Figure 7.

At 702, a first electrode is disposed proximate a frame having a cavity. As one example, the frame may be formed of an electrically conductive material, with the first electrode integrally formed with the frame (e.g., the frame defines, provides, or acts as the first electrode). As another example, the frame may be made of an insulative material, and the first electrode may be mounted to, affixed to, or otherwise disposed on a surface of the frame.

At 704, a dielectric plate is positioned a distance from the first electrode as discussed herein to define a volume. The volume is configured to hold a gas used to emit light when the excimer lamp is operated. For example, in the illustrated embodiment, at 706, an o-ring is placed in a groove of the frame, and, at 708, the dielectric plate is mounted to the frame with the o-ring interposed between the dielectric plate and the frame.

At 710, a second electrode is positioned. The second electrode is oriented toward the dielectric plate, and the dielectric plate is interposed between the first electrode and the second electrode. In some embodiments, at 712, a conductive ink is silk screen or otherwise printed on the dielectric plate using conductive ink to form the second electrode. In some embodiments, at 714, the second electrode is formed with a conductive mesh (e.g., via laser cutting). At 716, the dielectric plate is secured to the frame with a band. The band is coupled to the frame via a notch extending circumferentially around a side of the frame.

At 718, the volume is filled with gas. With the volume filled with gas, the electrodes may be coupled to an electrical supply, and the excimer lamp may be used. Because the excimer lamp produced using the method 700 may be produced in a variety of shapes, the shape of the lamp may be tailored to its intended location of use.

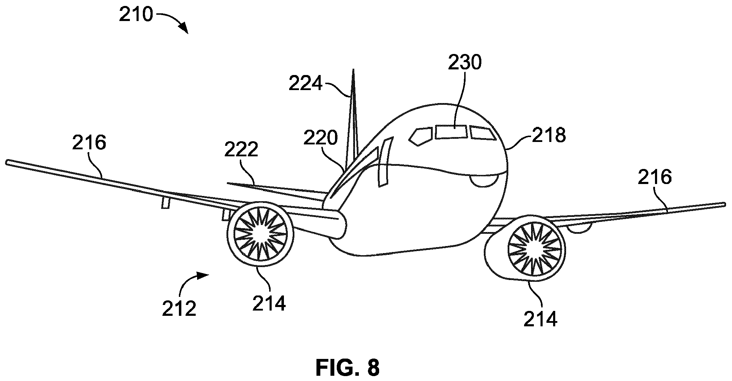

Figure 8 illustrates a perspective front view of an aircraft 210, according to an embodiment of the present disclosure. The aircraft 210 includes a propulsion system 212 that includes engines 214, for example. Optionally, the propulsion system 212 may include more engines 14 than shown. The engines 214 are carried by wings 216 of the aircraft 210. In other embodiments, the engines 214 may be carried by a fuselage 218 and/or an empennage 220. The empennage 220 may also support horizontal stabilizers 222 and a vertical stabilizer 224.

The fuselage 218 of the aircraft 210 defines an internal cabin 230, which includes a flight deck or cockpit, one or more work sections (for example, galleys, personnel carry-on baggage areas, and the like), one or more passenger sections (for example, first class, business class, and coach sections), one or more lavatories, and/or the like. The internal cabin 230 includes one or more lavatory systems, lavatory units, or lavatories, as described herein.

Alternatively, instead of an aircraft, embodiments of the present disclosure may be used with various other vehicles, such as automobiles, buses, locomotives and train cars, watercraft, and the like. Further, embodiments of the present disclosure may be used with respect to fixed structures, such as commercial and residential buildings.

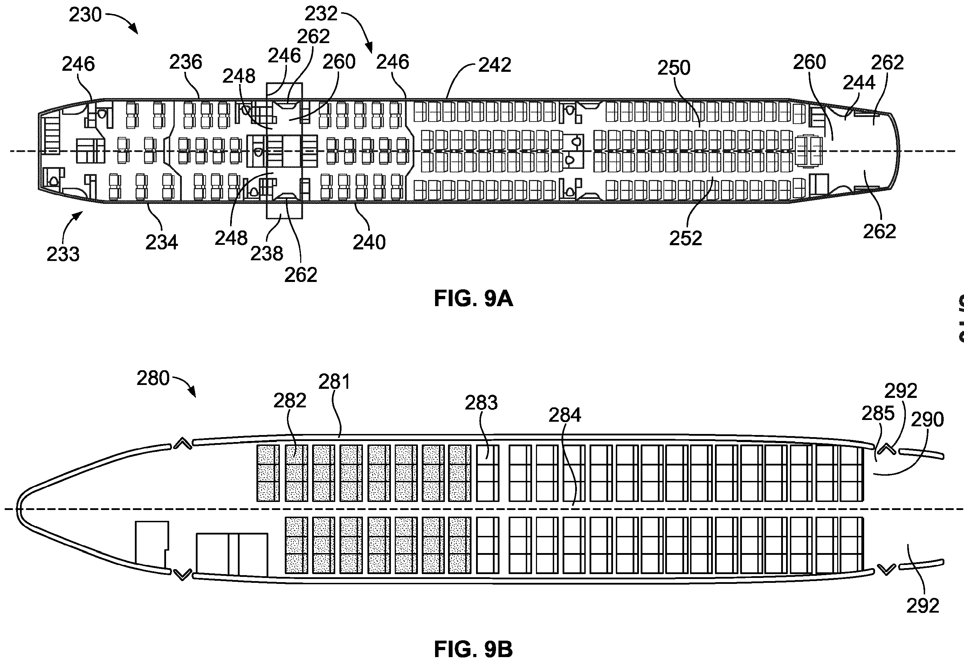

Figure 9A illustrates a top plan view of an internal cabin 230 of an aircraft, according to an embodiment of the present disclosure. The internal cabin 230 may be within the fuselage 232 of the aircraft, such as the fuselage 218 of Figure 8. For example, one or more fuselage walls may define the internal cabin 230. The internal cabin 230 includes multiple sections, including a front section 233, a first class section 234, a business class section 236, a front galley station 238, an expanded economy or coach section 240, a standard economy of coach section 242, and an aft section 244, which may include multiple lavatories and galley stations. It is to be understood that the internal cabin 230 may include more or less sections than shown. For example, the internal cabin 230 may not include a first class section, and may include more or less galley stations than shown. Each of the sections may be separated by a cabin transition area 246, which may include class divider assemblies between aisles 248.

As shown in Figure 9A, the internal cabin 230 includes two aisles 250 and 252 that lead to the aft section 244. Optionally, the internal cabin 230 may have less or more aisles than shown. For example, the internal cabin 230 may include a single aisle that extends through the center of the internal cabin 230 that leads to the aft section 244.

The aisles 248, 250, and 252 extend to egress paths or door passageways 260. Exit doors 262 are located at ends of the egress paths 260. The egress paths 260 may be perpendicular to the aisles 248, 250, and 252. The internal cabin 230 may include more egress paths 260 at different locations than shown. The portable sanitizing system 100 shown and described with respect to Figures 1-18 may be used to sanitize various structures within the internal cabin 230, such as passenger seats, monuments, stowage bin assemblies, components on and within lavatories, galley equipment and components, and/or the like.

Figure 9B illustrates a top plan view of an internal cabin 280 of an aircraft, according to an embodiment of the present disclosure. The internal cabin 280 is an example of the internal cabin 230 shown in Figure 8. The internal cabin 280 may be within a fuselage 281 of the aircraft. For example, one or more fuselage walls may define the internal cabin 280. The internal cabin 280 includes multiple sections, including a main cabin 282 having passenger seats 283, and an aft section 285 behind the main cabin 282. It is to be understood that the internal cabin 280 may include more or less sections than shown.

The internal cabin 280 may include a single aisle 284 that leads to the aft section 285. The single aisle 284 may extend through the center of the internal cabin 280 that leads to the aft section 285. For example, the single aisle 284 may be coaxially aligned with a central longitudinal plane of the internal cabin 280.

The aisle 284 extends to an egress path or door passageway 290. Exit doors 292 are located at ends of the egress path 290. The egress path 290 may be perpendicular to the aisle 284. The internal cabin 280 may include more egress paths than shown. The portable sanitizing system 100 shown and described with respect to Figures 1-18 may be used to sanitize various structures within the internal cabin 230, such as passenger seats, monuments, stowage bin assemblies, components on and within lavatories, galley equipment and components, and/or the like.

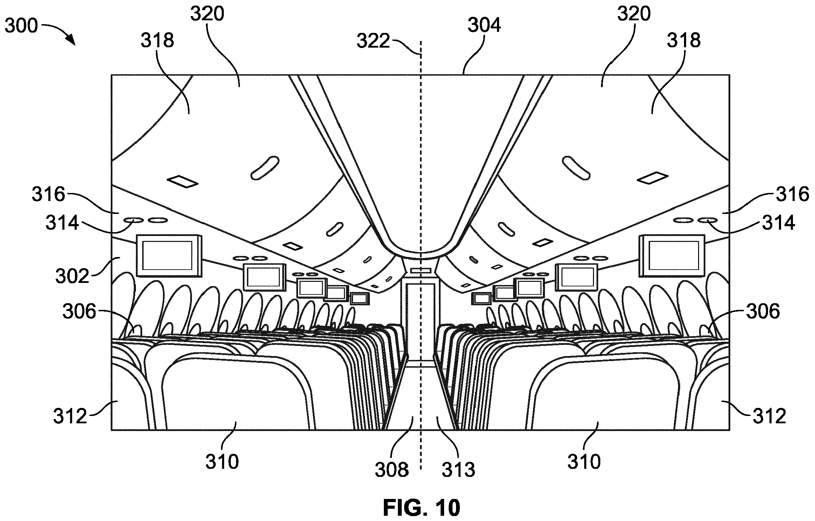

Figure 10 illustrates a perspective interior view of an internal cabin 300 of an aircraft, according to an embodiment of the present disclosure. The internal cabin 300 includes outboard walls 302 connected to a ceiling 304. Windows 306 may be formed within the outboard walls 302. A floor 308 supports rows of seats 310. As shown in Figure 10, a row 312 may include two seats 310 on either side of an aisle 313. However, the row 312 may include more or less seats 310 than shown. Additionally, the internal cabin 300 may include more aisles than shown.

Passenger service units (PSUs) 314 are secured between an outboard wall 302 and the ceiling 304 on either side of the aisle 313. The PSUs 314 extend between a front end and rear end of the internal cabin 300. For example, a PSU 314 may be positioned over each seat 310 within a row 312. Each PSU 314 may include a housing 316 that generally contains vents, reading lights, an oxygen bag drop panel, an attendant request button, and other such controls over each seat 310 (or groups of seats) within a row 312.

Overhead stowage bin assemblies 318 are secured to the ceiling 304 and/or the outboard wall 302 above and inboard from the PSU 314 on either side of the aisle 313. The overhead stowage bin assemblies 318 are secured over the seats 310. The overhead stowage bin assemblies 318 extend between the front and rear end of the internal cabin 300. Each stowage bin assembly 318 may include a pivot bin or bucket 320 pivotally secured to a strongback (hidden from view in Figure 10). The overhead stowage bin assemblies 318 may be positioned above and inboard from lower surfaces of the PSUs 314. The overhead stowage bin assemblies 318 are configured to be pivoted open in order to receive passenger carry-on baggage and personal items, for example.

As used herein, the term "outboard" means a position that is further away from a central longitudinal plane 322 of the internal cabin 300 as compared to another component. The term "inboard" means a position that is closer to the central longitudinal plane 322 of the internal cabin 300 as compared to another component. For example, a lower surface of a PSU 314 may be outboard in relation to a stowage bin assembly 318.

The excimer lamps discussed herein may be used to sanitize various structures shown within the internal cabin 300.

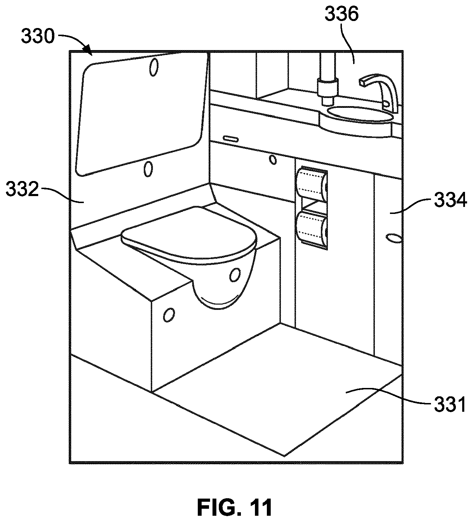

Figure 11 illustrates a perspective internal view of a lavatory 330 within an internal cabin of a vehicle, such as any of the internal cabins described herein. The lavatory 330 is an example of an enclosed space, monument or chamber, such as within the internal cabin a vehicle. The lavatory 330 may be onboard an aircraft, as described above. Optionally, the lavatory 330 may be onboard various other vehicles. In other embodiments, the lavatory 330 may be within a fixed structure, such as a commercial or residential building. The lavatory 330 includes a base floor 331 that supports a toilet 332, cabinets 334, and a sink 336 or wash basin. The lavatory 330 may be arranged differently than shown. The lavatory 330 may include more or less components than shown. The excimer lamps discussed herein may be used to sanitize the various structures, components, and surfaces within the lavatory 330.

Further, the disclosure comprises examples according to the following clauses:
Clause 1. An excimer lamp (100) comprising:
   a first electrode (110);
   a dielectric plate (120) having a first side (122) and a second side (124) opposite the first side (122), the dielectric plate (120) spaced a distance from the first electrode (110) to define a volume configured to hold a gas, the first side (122) of the dielectric plate (120) oriented toward the first electrode (110); and
   a second electrode (130) oriented toward the dielectric plate (120), wherein the dielectric plate (120) is interposed between the first electrode (110) and the second electrode (130).
Clause 2. The excimer lamp (100) of Clause 1, further comprising a frame (140) configured to support the dielectric plate (120).
Clause 3. The excimer lamp (100) of Clause 2, wherein the frame (140) comprises a groove (144) extending circumferentially around the frame (140), and the excimer lamp (100) comprises an o-ring (160) disposed in the groove (144) and interposed between the dielectric plate (120) and the frame (140).
Clause 4. The excimer lamp (100) of Clause 3, wherein the frame (140) further comprises a recessed surface (146) and a projection (148), the recessed surface (146) located proximate the groove (144), the projection (148) comprising a land and extending from the recessed surface (146) toward the dielectric plate (120).
Clause 5. The excimer lamp (100) of Clause 2, 3, or 4, wherein the frame (140) comprises a notch extending circumferentially around a side (143) of the frame (140), and the excimer lamp (100) comprises a band (170) mounted to the frame (140) in the notch, the band (170) securing the dielectric plate (120) to the frame (140).
Clause 6. The excimer lamp (100) of any one of Clauses 2-5, wherein the frame (140) is formed of an electrically conductive material and the first electrode (110) is integrally formed with the frame (140).
Clause 7. The excimer lamp (100) of any one of Clauses 2-6, wherein the frame (140) is formed of an electrically insulative material, and the first electrode (110) is supported by the frame (140).
Clause 8. The excimer lamp (100) of any one of Clauses 1-7, wherein the second electrode (130) is formed from a silk screened conductive ink.
Clause 9. The excimer lamp (100) of any one of Clauses 1-8, wherein the second electrode (130) is formed of a conductive mesh.
Clause 10. The excimer lamp (100) of any one of Clauses 1-9, wherein the excimer lamp (100) has a disc shape.
Clause 11. The excimer lamp (100) of any one of Clauses 1-10, wherein the dielectric plate (120) and the second electrode (130) have corresponding non-planar surfaces with a uniform offset distance.
Clause 12. The excimer lamp (100) of any one of Clauses 1-11, wherein the dielectric plate (120) and the second electrode (130) have a concave shape corresponding to an object to be irradiated.
Clause 13. An excimer lamp (100) comprising:
   a frame (140) made of a conductive material to define a first electrode (110), the frame (140) having a cavity (142);
   a dielectric plate (120) having a first side (122) and a second side (124) opposite the first side (122), the dielectric plate (120) spaced a distance from the frame (140) to define a volume configured to hold a gas, the first side (122) of the dielectric plate (120) oriented toward the frame (140); and
   a second electrode (130) oriented toward the dielectric plate (120), wherein the dielectric plate (120) is interposed between the frame (140) and the second electrode (130).
Clause 14. The excimer lamp (100) of Clause 13, wherein the frame (140) comprises a groove (144) extending circumferentially around the frame (140), and the excimer lamp (100) comprises an o-ring (160) disposed in the groove (144) and interposed between the dielectric plate (120) and the frame (140).
Clause 15. The excimer lamp (100) of Clause 13 or 14, further comprising a reflector mounted to a surface of the cavity (142) of the frame (140).
Clause 16. The excimer lamp (100) of Clause 13, 14, or 15, wherein the frame (140) comprises a notch extending circumferentially around a side of the frame (140), and the excimer lamp (100) comprises a band (170) mounted to the frame (140) in the notch, the band (170) securing the dielectric plate (120) to the frame (140).
Clause 17. The excimer lamp (100) of any one of Clauses 13-16, wherein a ground conductor is coupled to the frame (140).
Clause 18. The excimer lamp (100) of any one of Clauses 13-17, wherein a positive conductor is coupled to the second electrode (130).
Clause 19. The excimer lamp (100) of any one of Clauses 13-18, further comprising a protective plate located proximate the second electrode (130), wherein the second electrode (130) is interposed between the dielectric plate (120) and the protective plate.
Clause 20. An excimer lamp (100) comprising:
   a frame (140) made of an insulative material, the frame (140) having a cavity (142);
   a first electrode (110) mounted to the frame (140);
   a dielectric plate (120) having a first side (122) and a second side (124) opposite the first side (122), the dielectric plate (120) spaced a distance from the first electrode (110) to define a volume configured to hold a gas, the first side (122) of the dielectric plate (120) oriented toward the frame (140); and
   a second electrode (130) oriented toward the dielectric plate (120), wherein the dielectric plate (120) is interposed between the first electrode (110) and the second electrode (130).

While various spatial and directional terms, such as top, bottom, lower, mid, lateral, horizontal, vertical, front and the like can be used to describe embodiments of the present disclosure, it is understood that such terms are merely used with respect to the orientations shown in the drawings. The orientations can be inverted, rotated, or otherwise changed, such that an upper portion is a lower portion, and vice versa, horizontal becomes vertical, and the like.

As used herein, a structure, limitation, or element that is "configured to" perform a task or operation is particularly structurally formed, constructed, or adapted in a manner corresponding to the task or operation. For purposes of clarity and the avoidance of doubt, an object that is merely capable of being modified to perform the task or operation is not "configured to" perform the task or operation as used herein.

It is to be understood that the above description is intended to be illustrative, and not restrictive. For example, the above-described embodiments (and/or aspects thereof) can be used in combination with each other. In addition, many modifications can be made to adapt a particular situation or material to the teachings of the various embodiments of the disclosure without departing from their scope. While the dimensions and types of materials described herein are intended to define the parameters of the various embodiments of the disclosure, the embodiments are by no means limiting and are exemplary embodiments. Many other embodiments will be apparent to those of skill in the art upon reviewing the above description. The scope of the various embodiments of the disclosure should, therefore, be determined with reference to the appended claims, along with the full scope of equivalents to which such claims are entitled. In the appended claims and the detailed description herein, the terms "including" and "in which" are used as the plain-English equivalents of the respective terms "comprising" and "wherein." Moreover, the terms "first," "second," and "third," etc. are used merely as labels, and are not intended to impose numerical requirements on their objects. Further, the limitations of the following claims are not written in means-plus-function format and are not intended to be interpreted based on 35 U.S.C. § 112(f), unless and until such claim limitations expressly use the phrase "means for" followed by a statement of function void of further structure.

This written description uses examples to disclose the various embodiments of the disclosure, including the best mode, and also to enable any person skilled in the art to practice the various embodiments of the disclosure, including making and using any devices or systems and performing any incorporated methods. The patentable scope of the various embodiments of the disclosure is defined by the claims, and can include other examples that occur to those skilled in the art. Such other examples are intended to be within the scope of the claims if the examples have structural elements that do not differ from the literal language of the claims, or if the examples include equivalent structural elements with insubstantial differences from the literal language of the claims.

## Claims

1. An excimer lamp (100) comprising:
a first electrode (110);
a dielectric plate (120) having a first side (122) and a second side (124) opposite the first side (122), the dielectric plate (120) spaced a distance from the first electrode (110) to define a volume configured to hold a gas, the first side (122) of the dielectric plate (120) oriented toward the first electrode (110); and
a second electrode (130) oriented toward the dielectric plate (120), wherein the dielectric plate (120) is interposed between the first electrode (110) and the second electrode (130).

2. The excimer lamp (100) of Claim 1, further comprising a frame (140) configured to support the dielectric plate (120).

3. The excimer lamp (100) of Claim 2, wherein the frame (140) comprises a groove (144) extending circumferentially around the frame (140), and the excimer lamp (100) comprises an o-ring (160) disposed in the groove (144) and interposed between the dielectric plate (120) and the frame (140).

4. The excimer lamp (100) of Claim 3, wherein the frame (140) further comprises a recessed surface (146) and a projection (148), the recessed surface (146) located proximate the groove (144), the projection (148) comprising a land and extending from the recessed surface (146) toward the dielectric plate (120).

5. The excimer lamp (100) of Claim 2, 3, or 4, wherein the frame (140) comprises a notch extending circumferentially around a side (143) of the frame (140), and the excimer lamp (100) comprises a band (170) mounted to the frame (140) in the notch, the band (170) securing the dielectric plate (120) to the frame (140).

6. The excimer lamp (100) of any one of Claims 2-5, wherein the frame (140) is formed of an electrically conductive material and the first electrode (110) is integrally formed with the frame (140).

7. The excimer lamp (100) of any one of Claims 2-6, wherein the frame (140) is formed of an electrically insulative material, and the first electrode (110) is supported by the frame (140).

8. The excimer lamp (100) of any one of Claims 1-7, wherein the second electrode (130) is formed from a silk screened conductive ink.

9. The excimer lamp (100) of any one of Claims 1-8, wherein the second electrode (130) is formed of a conductive mesh.

10. The excimer lamp (100) of any one of Claims 1-9, wherein the excimer lamp (100) has a disc shape.

11. The excimer lamp (100) of any one of Claims 1-10, wherein the dielectric plate (120) and the second electrode (130) have corresponding non-planar surfaces with a uniform offset distance.

12. The excimer lamp (100) of any one of Claims 1-11, wherein the dielectric plate (120) and the second electrode (130) have a concave shape corresponding to an object to be irradiated.

13. An excimer lamp (100) comprising:
a frame (140) made of a conductive material to define a first electrode (110), the frame (140) having a cavity (142);
a dielectric plate (120) having a first side (122) and a second side (124) opposite the first side (122), the dielectric plate (120) spaced a distance from the frame (140) to define a volume configured to hold a gas, the first side (122) of the dielectric plate (120) oriented toward the frame (140); and
a second electrode (130) oriented toward the dielectric plate (120), wherein the dielectric plate (120) is interposed between the frame (140) and the second electrode (130).

14. The excimer lamp (100) of Claim 13, wherein the frame (140) comprises a groove (144) extending circumferentially around the frame (140), and the excimer lamp (100) comprises an o-ring (160) disposed in the groove (144) and interposed between the dielectric plate (120) and the frame (140).

15. The excimer lamp (100) of Claim 13 or 14, further comprising a reflector mounted to a surface of the cavity (142) of the frame (140).
